# EUROPÄISCHE PATENTSCHRIFT

(11) **EP 1 523 945 B1**
(45) Veröffentlichungstag und Bekanntmachung des Hinweises auf die Patenterteilung: **21.02.2007**
(21) Anmeldenummer: 04031018.7
(22) Anmeldetag: 30.10.2000
(51) Int. Cl.: A61B 17/12

(54) **Modul aus elektrolytisch korrodierbaren Formteilen und elektrolytisch nicht korrodierbaren Abschnitten von Occlusionswendeln sowie Formteil**
Module comprising electrolytically corrodible and non-corrodible sections for occlusion coils and and corrodible section
Module comprenant des pièces corrodables et non corrodables pour éléments hélicoidaux d'occlusion et pièce corrodable

(30) Priorität: 30.10.1999 DE 19952387; 09.03.2000 DE 10010840
(43) Veröffentlichungstag der Anmeldung: 20.04.2005
(62) Teilanmeldung aus: 00977439.9
(73) Patentinhaber: Dendron GmbH, 44799 Bochum (DE)
(72) Erfinder: Monstadt, Hermann, Dr., 44797 Bochum (DE)
(74) Vertreter: Thiel, Christian

(56) Entgegenhaltungen:
- WO-A-91/13592
- WO-A-99/09894
- US-A- 5 624 449
- US-A- 5 733 329
- US-A- 5 941 888

## Beschreibung

Die Erfindung betrifft ein Modul, welches geeignet ist, durch Aneinanderfügung mit weiteren Modulen eine zur Implantation in Blutgefäße, insbesondere Aneurysmen, bestimmte Occlusionswendel auszubilden, wobei sich das Modul aus aneinandergefügten elektrolytisch nicht korrodierbaren Abschnitten und elektrolytisch korrodierbaren Stellen zusammensetzt und wobei die elektrolytisch korrodierbaren Stellen als Formteile vorliegen, sowie ein Formteil. Deartige Module und Formteile sind aus US-A-5 941 888 bekannt.

Der Einsatz endovaskulärer Techniken zur Occlusion von Körperhohlräumen oder Gefäßen wie Arterien, Venen, Eileitern oder vaskulären Fehlbildungen wie z. B. vaskulärer Aneurysmen ist bekannter Stand der Technik. Die Occlusionswendel wird dabei in der Regel mit Hilfe eines endovaskulären Führungsdrahtes durch einen Katheter in den zu occludierenden Hohlraum eingeführt und deponiert.

Die zur Deponierung notwendige Abtrennung der Occlusionswendel gestaltet sich dabei technisch besonders problematisch, denn die hierzu notwendige Vorrichtung muß einerseits möglichst klein gehalten sein, um durch den geringen Durchmesser des Katheters an die Zielorte geführt werden zu können, andererseits muß sie eine zuverlässige Abtrennung der Occlusionswendel bewirken, da es sonst bei Rückführung des Katheters zu einer ungewollten Entfernung der Occlusionswendel aus dem zu occludierenden Hohlraum und somit zu Verletzung und/oder Ruptur der Hohlraum- oder Gefäßwandung kommen kann.

Mechanische Verfahren zur Abtrennung der Occlusionswendel von der Einführhilfe sind zwar mit einem geringen Zeitaufwand verbunden, die technisch bedingte Starrheit der Verbindung von Occlusionswendel und Einführhilfe erschwert jedoch die Implantateinbringung. Zudem birgt die durch die Starrheit bedingte geringe Belastbarkeit der Verbindung ein nicht unerhebliches Risiko der vorzeitigen Trennung von Einführhilfe und Occlusionsimplantat. Weiterhin muß bei der mechanischen Trennung von Einführdraht und Occlusionswendel Energie übertragen werden (in der Regel durch Rotation des Einführdrahtes), durch die auch das Implantat aus der gewünschten Position verlagert werden kann.

Die elektrolytische Ablösung von Edelstahl-Drahtspitzen im Blut während der Transkatheter Elektrokoagulation von Blutgefäßen oder Fehlbildungen derselben, wurde zum ersten mal 1979 durch Thompson et al und McAlister et al beschrieben (Radiology 133:335-340, November 1979, AJR 132:998-1000, Juni 1979).

Darauf aufbauend beschreibt die EP 0 484 468 eine Vorrichtung zur Implantation einer Occlusionswendel, basierend auf der elektrolytisch korrodierbaren Ausbildung der Drahtspitze des Führungsdrahtes an der Verbindung zwischen Führungsdraht und Occlusionswendel. Diese Vorrichtung nutzt zwar auf elegante Weise die zur Elektrothrombierung an die als Anode dienende Occlusionswendel angelegte elektrische Spannung für die gleichzeitige Abtrennung der Drahtspitze und der daran befindlichen Occlusionswendel, sie weist jedoch ebenso wie die vorgenannte mechanische Abtrennung den Nachteil auf, daß nur Implantate vordefinierter Länge abgetrennt werden können. Es muß also in der Regel vor der unmittelbaren Implantateinbringung anhand der Größe des zu occludierenden Hohlraumes die Länge, d. h. longitudinale Ausdehnung der einzusetzenden Occlusionswendel vordefiniert werden. Da die unregelmäßige Ausbildung der zu occludierenden Körperhohlräume jedoch die Einschätzung der zur Füllung notwendigen Länge der einzusetzenden Occlusionswendel erschwert, kann es dazu kommen, daß zu lange oder zu kurze Occlusionswendeln in den zu occludierenden Hohlraum eingeführt werden, was eine unvollständige Occludierung einerseits bzw. der Beschädigung oder Ruptur der Wandung des zu occludierenden Hohlraumes oder angrenzender Gefäße andererseits bedingen kann.

Ein weiterer Nachteil der elektrolytischen Ablösung der Führungsdrahtspitze ist die Tatsache, daß zur Ausbildung des Führungsdrahtes nur Materialien eingesetzt werden können, welche hohe Stabilität aufweisen, damit die sichere Führung des einzuführenden Occlusionsdrahtes durch den Führungsdraht möglich ist. Die Werkstoffauswahl für die Ausbildung der elektrolytischen Ablösestelle ist daher sehr eingeschränkt.

Bei den im Stand der Technik vorkommenden Vorrichtungen zur elektrolytischen Ablösung von Occlusionswendeln sind Occlusionswendel und Führungsdraht nicht aus einem Stück gefertigt, sondern in der Regel mechanisch miteinander verbunden. Diese Ausbildung birgt den Nachteil, daß zur Gewährleistung ausreichender Stabilität im proximalen Bereich des Führungsdrahtes und zwecks Ermöglichung der elektrolytisch korrosiven Auflösung der Drahtspitze im distalen Bereich des Drahtes der Führungsdraht mittels aufwendiger Schleifverfahren zur Spitze hin verjüngt werden muß. Dieser korrodierbare Bereich der Drahtspitze des Führungsdrahtes an der Verbindungsstelle zwischen Führungsdraht und Occlusionswendel kann jedoch zur Gewährleistung ausreichender Stabilität der Verbindungsstelle einen gewissen Mindestdurchmesser von etwa 0,05 mm nicht unterschreiten, da er einer hohen Biegebeanspruchung unterliegt. Die die Verbindungsstelle zwischen Occlusionswendel und Führungsdraht darstellende korrodierbare Drahtspitze ist daher recht starr und benötigt zur elektrolytisch korrosiven Auflösung relativ lange Zeiten.

Die DE 44 45 715 C2 beschreibt die durch einen Laserstrahl, der mittels einer mitgeführten Lichtleitfaser auf die zu spaltende Stelle des Implantates fokussiert wird, herbeigeführte Abtrennung einer Occlusionswendel von der Einführhilfe. Diese Vorrichtung erlaubt es, anhand des Füllungszustandes des zu occludierenden Hohlraumes während des Eingriffes die zur Füllung des Hohlraumes optimale Länge der Occlusionswendel abzutrennen. So kann selbst bei Verwendung von hinsichtlich der Länge einheitlichen Occlusionswendeln die zur Füllung des Hohlraumes optimal geeignete Länge abgetrennt und deponiert werden. Die zur Anwendung dieser Vorrichtung notwendige Technologie ist jedoch derzeit noch sehr kostenintensiv.

Da der Stand der Technik bisher keine hinsichtlich Kostenaufwand und Sicherheit befriedigende Möglichkeit zur endovaskulären Deponierung von Occlusionswendeln in der jeweils optimalen Länge bietet, liegt der Erfindung die Aufgabe zugrunde, eine Vorrichtung bereitzustellen, die es auf möglichst kostengünstigem, effektivem und sicherem Wege erlaubt, Occlusionswendeln in der jeweils richtigen Länge in Körperhöhlen oder Gefäßen zu deponieren.

Diese Aufgabe wird erfindungsgemäß gelöst durch ein Modul gemäß Anspruch 1 sowie das Formteil gemäß Anspruch 14.

Die Integration einer Vielzahl von elektrolytisch korrodierbaren Stellen in die Occlusionswendel bietet gegenüber herkömmlichen Vorrichtungen zur elektrolytischen Ablösung von Occlusionswendeln den Vorteil, daß während eines Implantatvorganges nicht nur eine sondern mehrere Längen derselben Wendel nacheinander abgelöst und in den zu occludierenden Hohlraum eingebracht werden können. Das spart nicht nur Kosten und Zeit, sondern dient außerdem der weiteren Minimierung des Operationsrisikos.

Die Erfindung beruht auf Experimenten der Erfinder, die ergaben, daß bei Anlegen eines Stromes an eine erfindungsgemäßes Modul sich überraschenderweise spezifisch die dem distalen Ende des Katheters am nächsten liegende elektrolytisch korrodierbar ausgebildete Stelle der Occlusionswendel durch Elektrolyse löst. Diese Spezifität basiert wahrscheinlich darauf, daß einerseits die sich im Katheter befindenden elektrolytisch korrodierbaren Stellen der Occlusionswendel durch den Katheter vom ionischen Medium isoliert sind, also keiner Elektrolyse unterliegen können und andererseits die Stromdichte aufgrund des nach distal zunehmenden Widerstandes in der Occlusionswendel von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf.

Das erfindungsgemäße Modul zur Implantation von Occlusionswendeln kombiniert wie keine im Stand der Technik bekannte andere Vorrichtung die Vorteile der Occlusionseffektivität mit operativer Sicherheit und Kostengünstigkeit. Die während des Implantationsvorganges bestimmbare Implantatlänge verhindert, daß für den zu occludierenden Hohlraum eine zu kurze Occlusionswendel eingebracht wird, die zur Bildung eines für den zu occludierenden Raum ungenügend großen Thrombus führt. Weiterhin wird verhindert, daß eine hinsichtlich des zu occludierenden Hohlraumes zu lange Occlusionswendel eingeschoben wird, was die Gefahr von Verletzungen oder Rupturen des zu füllenden Hohlraumes oder angrenzender Gefäße minimiert. Es handelt sich zudem bei der elektrolytischen Abtrennung von Occlusionswendeln um eine erprobte Technik, deren Parameter weitgehend bestimmt sind. Letztlich weist die erfindungsgemäße Vorrichtung zur Implantation von Occlusionswendeln den Vorteil auf, daß für die Massenfertigung günstige Einheitslängen an Occlusionswendeln verwendet werden können. Dies stellt einen Kostenvorteil gegenüber den bei der herkömmlichen elektrolytischen oder mechanischen Abtrennung verwendeten Occlusionswendeln vorbestimmter Länge dar, weil dazu unterschiedlich lange Occlusionswendeln konfektioniert werden müssen, die dann beim Implantationsvorgang in ihrer Gesamtheit durch Abtrennung der Drahtspitze in den zu occludierenden Hohlraum eingebaut werden.

Da die elektrolytisch korrodierbaren Stellen der erfindungsgemäßen Vorrichtung einen Teil der Occlusionswendel selbst darstellen und zudem in Vielzahl vorliegen, unterliegen sie im Vergleich zu den herkömmlichen, starren elektrolytisch ausgebildeten Verbindungen zwischen Führungsdraht und Occlusionswendel einer wesentlichen geringeren Biegebeanspruchung während des Implantationsprozesses. Diese geringe Biegebeanspruchung ermöglicht den Einsatz von elektrolytisch korrodierbaren Stellen mit wesentlich geringeren Durchmessern als im Stand der Technik, was zu einer verbesserten und schnelleren elektrolytischen Ablösbarkeit der Occlusionswendel führt. Solche geringen Durchmesser von weniger als 0,5 mm sind durch dafür geeignete, z. B. mechanische Verfahren zu erreichen.

Ein weitere Vorteill der Ausbildung der elektrolytisch korrodierbaren Stellen der erfindungsgemäßen Vorrichtung in der Occlusionswendel selbst gegenüber der im Stand der Technik vorkommenden Ablösung der Führungsdrahtspitze ist die wesentlich größere Auswahl an Materialien, welche zur Ausbildung der korrodierbaren Ablösestellen eingesetzt werden können. Im Gegensatz zur herkömmlichen Ablösung der Führungsdrahtspitze, müssen die in der Occlusionswendel angesiedelten elektrolytisch korrodierbaren Stellen der erfindungsgemäßen Vorrichtung keine besonders große Stabilität aufweisen, es können daher auch weniger stabile, flexiblere Werkstoffe eingesetzt werden, sofern sie korrodierbar und körperverträglich sind.

Zweckmäßigerweise kann sich proximal an die Occlusionswendel eine als Führungsdraht ausgebildete Einführhilfe anschließen. Eine solche Ausführungsform hat den Vorteil, daß der Führungsdraht aus gegenüber der Occlusionswendel kostengünstigeren Materialien ausgebildet sein kann, zumal er nicht in Kontakt mit dem Körpergewebe kommt. Weiterhin wird die Ausgestaltung des Führungsdrahtes vorzugsweise so bestimmt, daß sie eine gute Steuerung der Occlusionswendel durch den Katheter erlaubt, was zur besseren Deponierbarkeit beiträgt.

Bei einer solchen Ausgestaltung der erfindungsgemäßen Vorrichtung sind Einführhilfe und Occlusionswendel vorzugsweise durch Löt- und/oder Klebe- und/oder Schweißvorgänge und/oder mechanische Verbindungen miteinander verbunden. Es handelt sich um im Stand der Technik bekannte Verbindungsverfahren, die sich durch Einfachheit und die Stabilität der so herbeigeführten Verbindung auszeichnen.

In einer weiteren Ausführungsform können Führungsdraht und Occlusionswendel der erfindungsgemäßen Vorrichtung als Teile des selben Drahtes ausgebildet sein. Diese Ausführungsform zeichnet sich durch besondere Stabilität aus und kann sehr kostengünstig sein, da vorgenannte Anbindungsverfahren des Führungsdrahtes mit der Occlusionswendel wegfallen.

In einer besonders vorteilhaften Ausführungsform der erfindungsgemäßen Vorrichtung ist die Occlusionswendel oder ein Teil derselben als Mikrowendel ausgebildet. Diese Ausbildung weist den Vorteil auf, daß eine erhöhte Oberfläche für die Thrombosierung bereitgestellt wird. Zum selben Zweck können auch andere Ausgestaltungen der Occlusionswendel eingesetzt werden, die die Oberfläche derselben vergrößern, so sind beispielsweise Ausgestaltungen denkbar, deren distales Ende sich aufzweigt.

Um eine möglichst schonende und effektive Ausfüllung des zu occludierenden Hohlraumes zu gewährleisten, ist eine Ausbildung der erfindungsgemäßen Vorrichtung vorteilhaft, bei der die Occlusionswendel oder ein Teil derselben unter elastischer Vorspannung steht, so daß sie nach Entlassung aus dem Katheter Windungen ausbildet. Diese Ausbildung erlaubt eine dichte und schonende Ausfüllung des zu occludierenden Hohlraumes, ohne daß die Occlusionswendel durch die Wandung des zu occludierenden Hohlraumes zur Ausbildung-solcher Windungen geformt werden muß, was das Risiko einer Wandungs-Ruptur vermindert. Es kommt dabei durch die elastische Vorspannung zur Ausbildung sekundärer Windungen.

Zweckmäßigerweise können die elektrolytisch nicht-korrodierbaren Abschnitte der Occlusionswendel eines oder mehrere der folgenden Materialien enthalten: Edelmetalle oder Edelmetall-Legierungen, korrosionsbeständige Keramikwerkstoffe, korrosionsbeständige Kunststoffe, vorzugsweise Platinmetall-Legierungen.

Ebenso bevorzugt ist eine Ausbildung der erfindungsgemäßen Vorrichtung, deren Occlusionswendel an den elektrolytisch korrodierbaren Stellen eines oder mehrere der folgenden Materialien enthält: Keramikwerkstoffe, Kunststoffe, Nichtedelmetalle oder Legierungen derselben, vorzugsweise rostfreies Stahl

Hierbei sind insbesondere die nicht rostenden Stähle der Typen AISI 301, 304 oder 316 bzw. Untergruppen dieser Typen geeignet.

Die für die Ausbildung des Occlusionswendel verwendeten Keramikwerkstoffe und Kunststoffe sind elektrisch leitfähig.

Erfindungsgemäß werden für die Ausbildung der elektrolytisch nicht-korrodierbaren Abschnitte an den Übergängen zu den elektrolytisch korrodierbaren Stellen Materialkombinationen gewählt, die dazu geeignet sind, Lokalelemente auszubilden. Auf diese Weise wird - unabhängig von der Verringerung des Durchmessers der korrodierbaren Stellen - die elektrolytische Ablösbarkeit der Occlusionswendel verbessert.

Hierbei eignen sich am besten Materialkombinationen, in denen zur Ausbildung der elektrolytisch korrodierbaren Stellen nicht rostende Stähle, vorzugsweise der Typen AISI 301, 304, 316 oder Untergruppen derselben, Ti oder TiNi-Legierungen oder Co-Basislegierungen mit einem oder mehreren der folgenden Edelmetalle bzw. Edelmetall-Legierungen: Pt, Pt-Metalle, Pt-Legierungen, Au-Legierungen oder Sn-Legierungen.

Der Einsatz der vorgenannten Materialien zur Ausbildung der elektrolytisch nicht-korrodierbaren Abschnitte und der elektrolytisch korrodierbaren Stellen der Occlusionswendel gewährleisten die spezifische elektrolytische Korrosion der Occlusionswendel an den dafür vorbestimmten Stellen.

Gemäß der Erfindung erfolgt die Ausbildung von elektrolytisch korrodierbaren Stellen derart, daß zu beiden Seiten Lokalelemente ausgebildet sind. Diese Ausführungsform der elektrolytisch korrodierbaren Stellen ist wesentlich korrosionsfreudiger und korrodiert daher wesentlich schneller als elektrolytisch korrodierbare Stellen, welche nur zu einer Seite ein Lokalelement ausbilden. Bevorzugt sind deshalb Materialkombinationen mit möglichst großem Abstand in der elektrochemischen Spannungsreihe. Auch dies ist ein Vorteil der erfindungsgemäßen Vorrichtung gegenüber den im Stand der Technik vorkommenden Vorrichtungen die zur Ablösung des Embolisierungsdrahtes die Spitze des Führungsdrahtes korrodieren, da in diesem Fall nur zu einer Seite, nämlich zur Seite des Embolisierungsdrahtes (in der Regel ein Platindraht) ein Lokalelement ausgebildet wird.

Die Form der elektrolytisch korrodierbaren Stellen ist zweckmäßigerweise unter funktionellen Gesichtspunkten ausgestaltet. So ist es in Hinsicht auf die Biegebeanspruchung vorteilhaft, die Form der elektrolytisch korrodierbaren Stellen an die Form der Occlusionswendel anzupassen und diese beispielsweise in die Windungen einer als Mikrowendel ausgebildeten Occlusionswendel zu integrieren. Andererseits hat die im wesentlichen gerade Ausbildung der elektrolytisch korrodierbaren Stellen den Vorteil, daß sie technisch unaufwendig ist. Zum Zwecke einer guten Verschieblichkeit der Occlusionswendel im Katheter ist dabei die Ausrichtung der im wesentlichen geraden elektrolytisch korrodierbaren Stellen in Längsachse der Occlusionswendel vorteilhaft.

Die elektrolytisch korrodierbaren Stellen der Occlusionswendel werden dabei durch Formteile gebildet, die zwischen die elektrolytisch nicht korrodierbaren Anteile der Occlusionswendel gefügt werden. Diese Ausführungsform hat den Vorteil, daß dabei eine besonders große Vielzahl verschiedener-Werkstoffe für die Ausbildung der elektrolytisch korrodierbaren Stellen und elektrolytisch nicht-korrodierbaren Abschnitte miteinander kombiniert werden können. Diese Ausführungsform weist weiterhin den Vorteil auf, daß die elektrolytisch korrodierbaren Stellen und die elektrolytisch nicht korrodierbaren Abschnitte modular und daher technisch unaufwendig zur Bildung von Occlusionswendeln variabler Längen aneinandergefügt werden können. Dies ist besonders einfach, wenn die elektrolytisch korrodierbaren Stellen und somit die sie bildenden Formteile im wesentlichen gerade ausgebildet sind.

Zweckmäßigerweise können die die elektrolytisch korrodierbaren Stellen bildenden Formteile mit den nicht-korrodierbaren Abschnitten durch Löt- und/oder Klebe- und/oder Schweißvorgänge verbunden sein. Ebenso können die die elektrolytisch korrodierbaren Stellen bildenden Formteile mechanisch an die elektrolytisch nicht korrodierbaren Abschnitte angefügt werden, z. B. durch Einspannung oder Einklemmung, sofern die elektrolytisch nicht korrodierbaren Abschnitte Ausnehmungen zur Aufnahme der Formteile aufweisen. Dies ist z.B der Fall bei elektrolytisch nicht korrodierbaren Abschnitten, die als einen inneren Hohlraum umschreibende Mikrowendeln ausgebildet sind. Die Formteile können so formschlüssig in diesen Hohlraum eingefügt und fixiert werden. Dabei kann es weiterhin zweckmäßig sein, wenn die die Formteile aufnehmenden Außenbereiche der als Mikrowendel ausgebildeten elektrolytisch nicht korrodierbaren Abschnitte verstärkt sind.

Es ist dabei besonders vorteilhaft, wenn die die elektrolytisch korrodierbaren Stellen bildenden Formteile durch Ätzen oder andere Verfahren vorkorrodiert sind, so daß sich ihr Durchmesser zur Mitte hin verjüngt. Die äußeren bzw. geschwächten Anteile der Formteile mit größerem Durchmesser werden dann durch beispielsweise artfremdes Verschweißen, mechanische Einbringung oder Verklebung an die elektrolytisch nicht korrodierbaren Abschnitte gefügt. Die Verbindung zwischen elektrolytisch korrodierbaren Stellen und elektrolytisch nicht korrodierbaren Abschnitten ist daher sehr stabil, wohingegen der sich infolge der Vorkorrosion zur Mitte des Formteils verjüngende Durchmesser zur guten elektrolytischen Ablösung der Occlusionswendel führt. Dabei ist für die Materialkombination von Platin-Legierungen oder Platin-Metallen als Werkstoff für die Ausbildung der elektrolytisch nicht korrodierbaren Abschnitte mit nichtrostendem Stahl als Werkstoff für die die elektrolytisch korrodierbaren Stellen bildenden Formteile die Verbindung durch artfremdes Verschweißen besonders bevorzugt. Für den Fachmann selbstverständlich ist auch die Möglichkeit der Vorkorrodierung der elektrolytisch korrodierbaren Stellen, wenn diese nicht aus Formteilen gebildet werden.

Es ist dabei zweckmäßig, die Formteile mit einer teilweisen Beschichtung eines Werkstoffs zu versehen, der in der Spannungsreihe über dem Material steht, welches die Formteile ausbildet. Diese Ausführungsform ist besonders vorteilhaft hinsichtlich elektrolytischen Korrodierbarkeit der elektrolytisch korrodierbaren Stellen, welche dort angesiedelt sind, wo die Beschichtung am Formteil fehlt. Als besonders vorteilhaft haben sich hierbei Beschichtungen von Zn oder Sn bzw. Legierungen dieser Metalle auf Formteilen aus nicht rostendem Stahl herausgestellt.

Die mechanische Anbringung der Formteile ist dabei besonders vorteilhaft, wenn die elektrolytisch korrodierbaren Stellen im wesentlichen gerade ausgebildet und entlang der Längsachse der Occlusionswendel angeordnet sein sollen. Die Aneinanderfügung der die Occlusionswendel bildenden Module (nicht elektrolytisch korrodierbare Abschnitte und elektrolytisch korrodierbare Stellen) ist in diesem Fall mit einem besonders niedrigen technischen Aufwand verbunden.

Zur weiteren Sicherung und Stabilisierung der Aneinanderfügung der einzelnen, die Occlusionswendel bildenden Module, kann auch eine Kombination der genannten Verfahren eingesetzt werden.

Die Flexibilität der Occlusionswendel ist auch bei mechanischer Aneinanderfügung aufgrund der Materialauswahl und aufgrund des geringen Durchmessers der die elektrolytisch korrodierbaren Stellen bildenden Formteile gewährleistet.

In einer Ausführung der erfindungsgemäßen Vorrichtung sind die die elektrolytisch korrodierbaren Stellen bildenden Formteile als Mikrosystembauteile ausgebildet. Diese können beispielsweise als längliche Mikrosystembauteile ausgebildet sein, deren Durchmesser sich zur Mitte hin verjüngt. Das Einfügen der Mikrosystembauteile erfolgt durch die genannten gängigen Verfahren. Die Verwendung solcher sich zur Mitte hin verjüngender Mikrosystemenbauteile weist dabei den Vorteil auf, daß die Bereiche des größten Durchmessers an die elektrolytisch nicht korrodierbaren Abschnitte gefügt werden und somit einen stabilen Zusammenhalt gewährleisten. Der sich verjüngende Bereich mit geringerem Durchmesser ist dagegen dem umgebenden Medium ausgesetzt und kann leicht elektrolytisch korrodiert werden. Auf diese Art und Weise können besonders geringe Durchmesser der elektrolytisch korrodierbaren Stellen erzielt werden.

Die Verjüngung des Durchmessers der elektrolytisch korrodierbaren Stellen zur Mitte hin ist hinsichtlich guter Korrodierbarkeit auch für andere Ausbildungen der elektrolytisch korrodierbaren Stellen zweckmäßig.

In einer weiteren Ausführungsform der erfindungsgemäßen Vorrichtung enthält die Occlusionswendel oder ein Teil derselben einen durchgängigen Drahtkern aus elektrolytisch korrodierbarem Material, welcher von einer in Längsachse in Abständen unterbrochenen Umhüllung aus elektrolytisch korrosionsbeständigem Material umgeben ist. Der Führungsdraht und der Kern der Occlusionswendel sind dabei vorzugsweise Teile desselben Drahtes. Diese Ausführungsform ist besonders kostengünstig, weil einerseits der zur Verbindung von Occlusionswendel und Einführhilfe notwendige Schweiß-, Löt- oder Klebevorgang entfällt und andererseits der Drahtkern aus in der Regel gegenüber den elektrolytisch nicht-korrodierbaren Materialien kostengünstigeren elektrolytisch korrodierbarem Material besteht, wohingegen nur geringe Mengen an gegenüber dem Drahtkern elektrolytisch schlechter korrodierbarem Material zur Beschichtung eingesetzt werden müssen.

Der Durchmesser der Occlusionswendel der erfindungsgemäßen Vorrichtung wird dabei zweckmäßigerweise so gewählt, daß er einerseits hinreichende Stabilität gewährleistet und andererseits die elektrolytisch korrodierbaren Stellen in situ elektrolytisch gut korrodierbar sind. Vorteilhaft in dieser Hinsicht sind Ausführungsformen mit an den elektrolytisch korrodierbaren Stellen vorliegenden Durchmessern der Occlusionswendel zwischen 0,01 bis 0,05 mm, vorzugsweise 0,02 bis 0,04 mm und besonders bevorzugt 0,03 mm. Die elektrolytisch nicht korrodierbaren Abschnitte der Occlusionswendel können dagegen auch größere Durchmesser aufweisen.

In einer weiteren vorteilhaften Ausführungsform ist die Spitze des Führungsdrahtes beispielsweise durch eine schlecht korrodierbare Materialbeschichtung oder Überzug mit einem Schrumpfschlauf isoliert, so daß sie nicht elektrolytisch korrodierbar ist.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln und/oder Tumorembolisation durch Thrombosierung bestimmt.

Die Erfindung wird nachfolgend beispielhaft anhand der in den Zeichnungen veranschaulichten Ausführungsbeispiele näher erläutert. Es stellen dar:

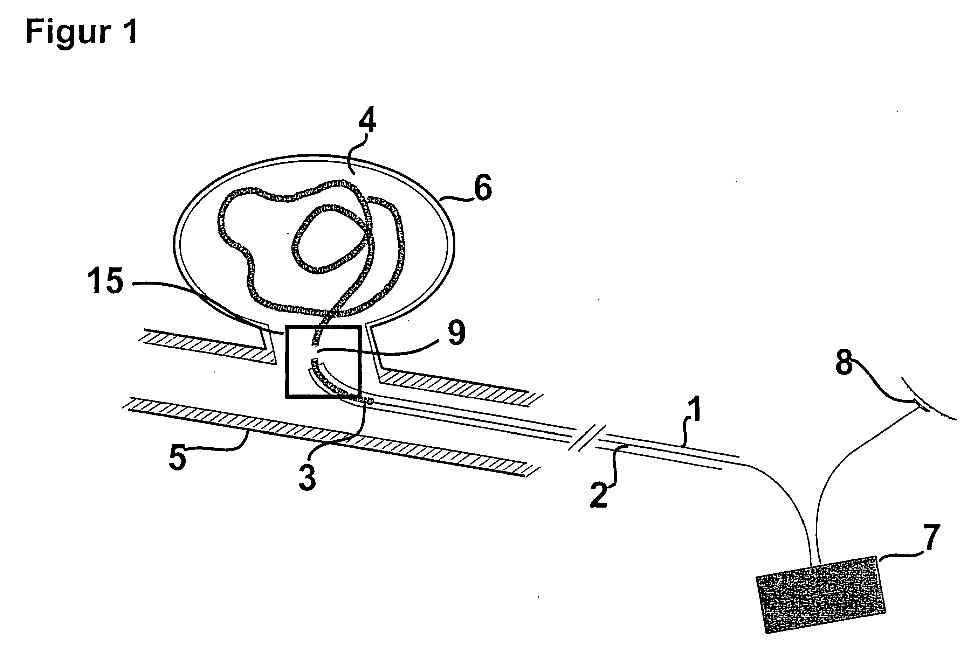

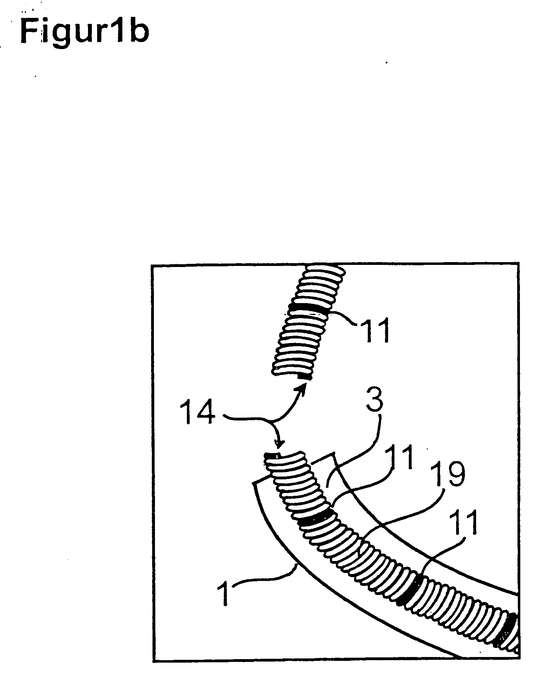

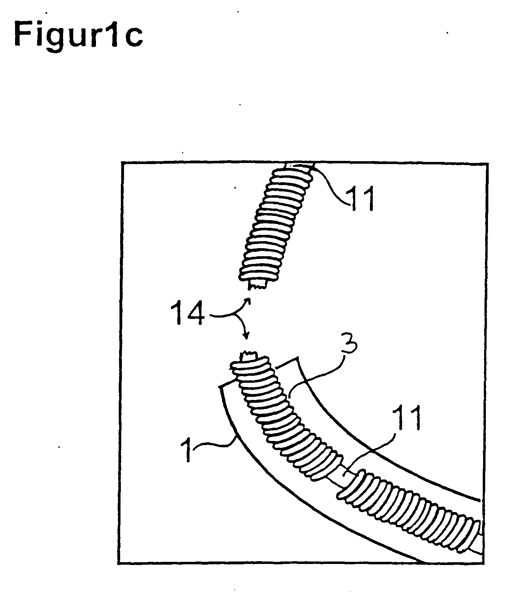

- Figur 1: die Vertikalansicht einer in ein Beerenaneurysma positionierten Mikrowendel 3 mit dazugehöriger Vorrichtung in vielfacher Vergrößerung;
- Figur 1 b und 1 c: die Ausschnitts-Vergrößerung 15 aus Figur 1 in dem Bereich der elektrolytisch korrodierten Stelle 14 der Mikrowendel 3 in zwei unterschiedlichen Ausbildungen der Mikrowendel 3;
- Figur 2a bis c: in gegenüber Figur 1 stärkerer Vergrößerung drei Möglichkeiten der Anordnung elektrolytisch korrodierbarer Stellen 11 und elektrolytisch nicht korrodierbarer Abschnitte 10 in der erfindungsgemäßen Mikrowendel 3;
- Figur 3a bis c: in gegenüber Figur 1 stärkerer Vergrößerung drei Möglichkeiten der Anordnung von elektrolytisch korrodierbarer Stellen 11 und elektrolytisch nicht korrodierbarer Abschnitte 10 in der erfindungsgemäßen Mikrowendel 3 mit entlang der Längsachse der Mikrowendel 3 angeordneten elektrolytisch korrodierbaren Stellen 11;
- Figur 4: Längsschnittansicht der Anbringung des Führungsdrahtes (2) an die Occlusionswendel.

In der Figur 1 ist mit 1 ein Katheter, insbesondere ein biegsam ausgebildeter Mikrokatheter bezeichnet. Durch den Mikrokatheter 1 wird die aus einer Platinmetall-Legierung gefertigte, mit elektrolytisch korrodierbaren Stellen 11 aus Edelstahl versehene als Mikrowendel ausgebildete Occlusionswendel 3 mit Hilfe des schweißtechnisch an die Mikrowendel 3 gefügten Führungsdrahtes 2 an den Eingang des Aneurysmas 6 positioniert. Da diese durch artfremdes Schweißen gefügte Verbindung zwischen Führungsdraht 2 und Mikrowendel 3 nicht zur elektrolytischen Ablösung der Mikrowendel 3 bestimmt ist und folglich nicht von besonders geringem Durchmesser sein muß, ist sie besonders stabil ausgebildet. Die Verwendung von nichtrostendem Stahl und Platin für Ausbildung des Führungsdrahtes einerseits bzw. der Occlusionswendel andererseits ist dabei besonders vorteilhaft, da der in Stahl enthaltene Nickel sich beim Verschweißen zu einer sehr glatten und stabilen Verbindung mit dem Platin fügt. Durch die in Längsachse des Mikrokatheters nach distal erfolgende Verschiebung der Führungshilfe 2 erfolgt die Einführung der Mikrowendel 3 in das Aneurysma 6, welche aufgrund ihrer elastischen Vorspannung nach Verlassen des Mikrokatheters 1 sekundäre Windungen 4 ausbildet. Durch die Längsverschieblichkeit von Führungsdraht 2 und Mikrowendel 3 im Mikrokatheter 1 wird eine an das Volumen des auszufüllenden Hohlraumes individuell angepaßte Länge der Mikrowendel 3 in diesen eingebracht. Anschließend wird mit Hilfe der Spannungsquelle 7, der auf der Körperoberfläche positionierten Kathode 8 und der als Anode dienenden im zu occludierenden Aneurysma 6 positionierten Mikrowendel 3 eine Spannung über einen Zeitraum von 0,1 - 20 Minuten angelegt. Dadurch wird die elektrolytische Abtrennung des sich im Blut befindenden Teils der Mikrowendel 3 an der dem distalen Katheterende nächstliegenden elektrolytisch korrodierbaren Stelle 9 aufgelöst. Figur 1 stellt eine Mikrowendel 3 dar, deren dem distalen Ende des Mikrokatheters 1 nächstliegende elektrolytisch korrodierbare Stelle 9 bereits elektrolytisch korrodiert wurde.

Die Figur 1b stellt in Ausschnittsvergrößerung der Figur 1 die dem distalen Ende des Mikrokatheters 1 nächstliegende elektrolytisch korrodierbare Stelle in korrodiertem Zustand 14 dar. Weitere, sich im Blut oder noch im Mikrokatheder 1 befindende elektrolytisch korrodierbare Stellen 11 liegen dagegen noch in intaktem Zustand vor. Die elektrolytisch korrodierbaren Stellen sind an die Form der Mikrowindungen 19 der Mikrowendel angepaßt.

Die Figur 1 c stellt ebenfalls in Ausschnittsvergrößerung der Figur 1 die dem distalen Ende des Mikrokatheters 1 nächstliegende elektrolytisch korrodierbare Stelle in korrodiertem Zustand 14 für eine Mikrowendel 3 mit im wesentlichen geraden elektrolytisch korrodierbaren Stellen 11 dar, die an der Längsachse der Mikrowendel 3 ausgerichtet sind.

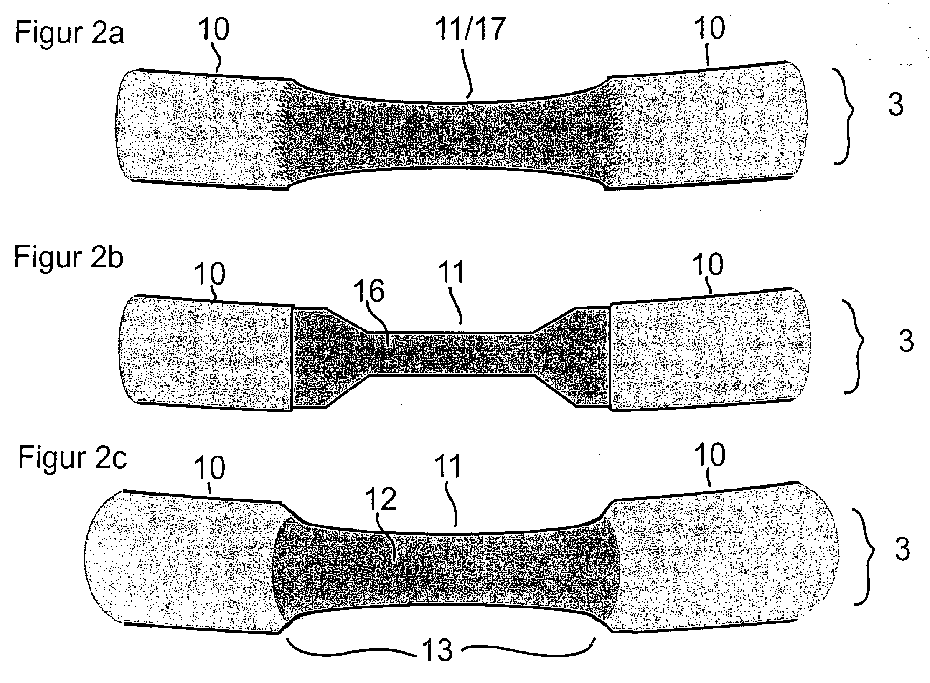

Die Figuren 2a bis c stellen in gegenüber Figur 1 stärkerer Vergrößerung einen Ausschnitt dreier unterschiedlicher Ausführungsformen der erfindungsgemäßen Mikrowendel 3 dar.

Die Figur 2a zeigt eine Mikrowendel 3 mit nicht-korrodierbaren Abschnitten 10 aus einer Platinmetall-Legierung, an die schweißtechnisch eine aus Edelstahl bestehende die elektrolytisch korrodierbare Stelle 11 bildendes Formteil 17 von 0,03 mm Durchmesser angefügt ist.

Die Figur 2b zeigt einen Ausschnitt aus einer erfindungsgemäßen Mikrowendel 3 mit einem Mikrosystembaustein 16 als elektrolytisch korrodierbare Stelle 11, welcher durch einen Klebevorgang zwischen die nicht elektrolytisch korrodierbaren Abschnitte 10 eingefügt ist.

Die Figur 2c stellt einen Ausschnitt einer einen Edelstahlkern 12 von 0,03 mm Durchmesser enthaltenden Mikrowendel 3 dar. Dieser Edelstahlkern 12 ist von einer Beschichtung aus elektrolytisch-korrosionsbeständigem Material 13 umgeben, die in regelmäßigen Abständen mit Unterbrechungen versehen ist, an denen der Edelstahlkern 12 von außen zugänglich ist und dementsprechend eine elektrolytisch korrodierbare Stelle 11 ausbildet.

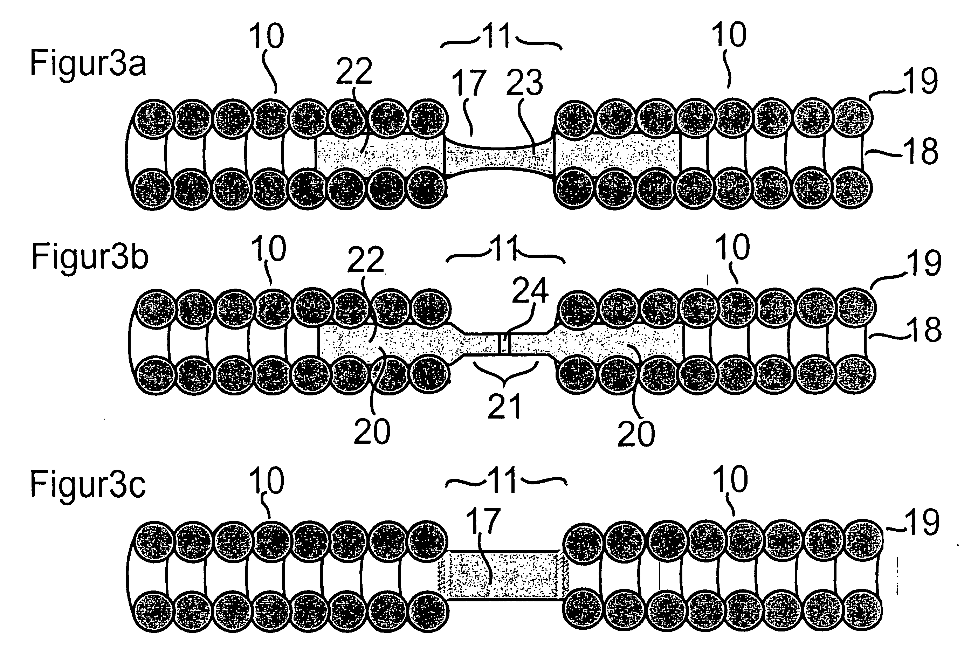

Die Figuren 3a-c stellen in gegenüber Figur 1 stärkerer Vergrößerung einen Ausschnitt dreier unterschiedlicher Ausführungsformen der erfindungsgemäßen Mikrowendel 3 mit in der Längsachse der Mikrowendel 3 ausgerichteten elektrolytisch korrodierbaren Stellen 11 dar.

Die Figur 3a zeigt eine Mikrowendel 3, mit einem im wesentlichen geraden Formteil 17 aus nicht rostendem Stahl, welches formschlüssig in den Innenraum 18 der Mikrowindungen 19 aus Platindraht eingefügt ist. Die modulare Aneinanderfügung von elektrolytisch nicht korrodierbaren Abschnitten 10 aus Platindraht-Mikrowindungen 19 und im wesentlichen geraden Formteilen 17 führt in regelmäßigen Abständen dort, wo die im wesentlichen geraden Formteile 17 nicht von den Platinmetall-Mikrowindungen 19 umgeben und somit von außen zugänglich sind, zur Ausbildung von elektrolytisch korrodierbaren Stellen 11. Die nicht korrodierbaren Abschnitte 10 der Mikrowendel 3 werden dagegen durch den in Mikrowindungen 19 gewundenen Platindraht gebildet, welcher sich jeweils mechanisch formschlüssig zu beiden Seiten um die im wesentlichen gerade ausgebildeten Edelstahlformteile 17 schließt. Das Formteil 17 ist von einer Sn-Schicht 22 umgeben, welche in der Mitte infolge der Vorkorrodierung abgelöst ist. Infolge dessen ist der der elektrolytisch korrodierbare Stelle 11 ausbildende vorkorrodierte Mittelteil 23 des Formteils 17 der elektrolytischen Korrosion besonders gut zugänglich, da er einen sehr geringen Durchmesser aufweist und zu beiden Seiten infolge der Sn-Beschichtung Lokalelemente ausbildet.

Die Figur 3b stellt ebenfalls eine modular aufgebaute Mikrowendel 3 dar, in der sich die Mikrowindungen 19 des Platindrahtes formschlüssig um die Enden eines aus Edelstahl gefertigten Mikrosystembausteins 16 schließen und so die elektrolytisch nicht korrodierbaren Abschnitte 10 bilden, zwischen denen die freiliegenden Abschnitte des Mikrosystembausteins 16 die elektrolytisch korrodierbare Stelle 11 ausbilden. Die Einfügung der Abschnitte des Mikrosystembausteins 16 mit größerem Durchmesser 20 in den Innenraum 18 der Platindrahtmikrowindungen 19 gewährleistet eine stabile Fixierung der modularen Elemente aneinander. Die Ausbildung der elektrolytisch korrodierbaren Stelle 11 durch den verjüngten Abschnitt 21 des Mikrosystembausteins 16 mit geringerem Durchmessers 21 ermöglicht dagegen ein großes Maß an Flexibilität sowie vorteilhaft gute Korrodierbarkeit der elektrolytisch korrodierbaren Stelle 11.

Diese Korrodierbarkeit wird verstärkt durch den Aufbau des Mikrosystembausteins, welcher zum großen Teil aus einer Sn-Legierung 22 besteht und lediglich in der sich verjüngenden Mitte ein Mikroablöseelement 24 enthält, welches aus nicht rostendem Stahl besteht und eine extrem kleine elektrolytisch korrodierbare Stelle ausbildet. Diese vorteilhafte Ausbildungsform ist extrem leicht korrodierbar und daher besonders gut ablösbar.

In Figur 3c ist alternativ der Ausschnitt aus einer erfindungsgemäßen Mikrowendel 3 mit einem im wesentlichen geraden die elektrolytisch korrodierbare Stelle 11 bildenden Formteil 17 aus Edelstahl gezeigt, das schweißtechnisch an die die nicht korrodierbaren Abschnitte 10 bildenden Mikrowindungen 19 aus Platindraht gefügt ist.

Die Elektronegativitäts-Unterschiede zwischen dem die korrodierbaren Stellen 11 ausbildenden Edelstahl und der die nicht-korrodierbaren Abschnitte 10 ausbildenden Platinmetall-Legierung bewirkt in einem ionischen Medium wie dem Blut bei Anlegen einer elektrischen Spannung die elektrolytische Auflösung der elektrolytisch korrodierbaren Stellen 11.

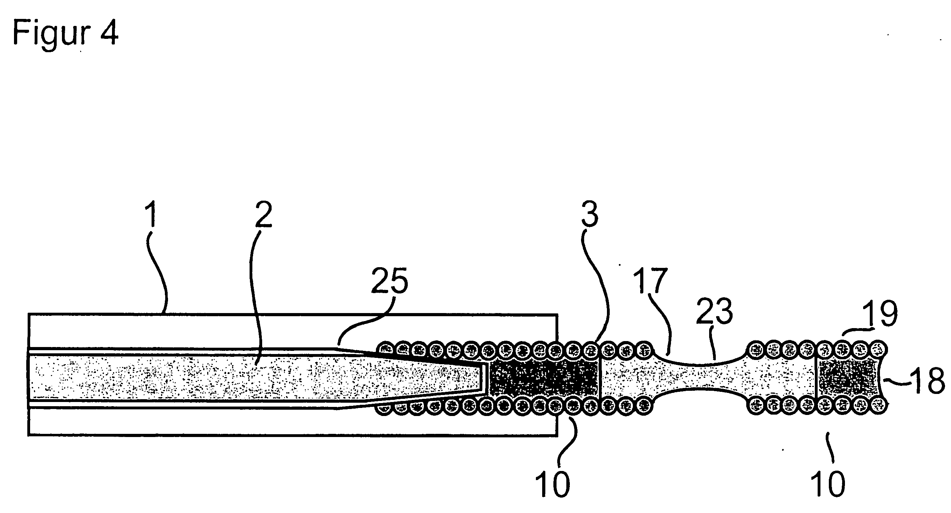

Die Figur 4 stellt einen Längsschnitt durch den Übergang zwischen dem Führungsdraht 2 und der Occlusionswendel 3 in Vergrößerung dar. In diesem Beispiel ist der Führungsdraht 2 aus stabilem nicht rostendem Stahl ausgebildet und von einer Beschichtung 25 umgeben, welche die Korrosion des Stahls verhindert. Diese Beschichtung kann entweder nicht leitend ausgebildet sein, in diesem Falle wird der elektrische Strom für das Ende des Mikrokatheters 1 an die Occlusionswendel 3 geleitet. Oder die Beschichtung ist nicht korrodierbar aber elektrisch leitend (beispielsweise eine Graphitbeschichtung) in welchem Fall der elektrische Strom auch über den Führungsdraht 2 in die Occlusionswendel 3 geleitet werden kann. In diesem Beispiel ist die Occlusionswendel 3 durch eine Anordnung von vorkorrodierten Formteilen 17 aus nicht rostendem Stahl gebildet, welche mechanisch und mit Hilfe von Verkleben in den Innenraum 18 von die elektrolytisch nicht korrodierbaren Stellen bildenden Platindraht Mikrowindungen 19 gefügt sind. In der Mitte vorkorrodierten Formteile 17 bilden aufgrund ihres geringen Durchmessers elektrolytisch besonders gut korrodierbare Stellen aus.

## Patentansprüche

1. Modul, welches geeignet ist, durch Aneinanderfügung mit weiteren Modulen eine zur Implantation in Blutgefäße, insbesondere Aneurysmen, bestimmte Occlusionswendel (3) auszubilden, wobei sich das Modul aus aneinandergefügten elektrolytisch nicht korrodierbaren Abschnitten (10) und elektrolytisch korrodierbaren stellen (11) zusammensetzt, die elektrolytisch korrodierbaren Stellen (11) als Formteile (17) vorliegen, und sich die Formteile (17) und die elektrolytisch nicht korrodierbaren Abschnitte (10) aus Materialkombinationen zusammensetzen, die zu beiden Seiten des Formteils (17) Lokalelemente ausbilden.

2. Modul nach Anspruch 1, **dadurch gekennzeichnet, daß** die elektrolytisch nicht korrodierbaren Abschnitte (10) Edelmetalle oder Edelmetall-Legierungen enthalten, vorzugsweise Platinmetall-Legierungen.

3. Modul nach Anspruch 1 oder 2, **dadurch gekennzeichnet, daß** die Formteile (17) Nicht-Edelmetalle oder Legierungen derselben, vorzugsweise nicht rostenden Stahl enthalten.

4. Modul nach Anspruch 3, **dadurch gekennzeichnet, daß** nicht rostender Stahl der Typen AISI 301, 304 oder 316 oder Untergruppen zur Ausbildung der elektrolytisch korrodierbaren Stellen (11) verwandt wird.

5. Modul nach einem der vorhergehenden Ansprüche 1-3, **dadurch gekennzeichnet, daß** folgende Materialkombinationen eingesetzt werden:
a) nicht rostende Stähle, vorzugsweise vom Typ AISI 301, 304, 316 oder Untergruppen für die Ausbildung der elektrolytisch korrodierbaren Stellen (11) mit Edelmetallen oder Edelmetall-Legierungen, vorzugsweise Pt oder Pt-Metallen, Pt-Legierungen, Au-Legierungen oder Sn-Legierungen zur Ausbildung der elektrolytisch nicht korrodierbaren Abschnitte (10);
b) Ti oder TiNi-Legierungen zur Ausbildung der elektrolytisch korrodierbaren Stellen (11) mit Edelmetallen oder Edelmetall-Legierungen, vorzugsweise Pt, Pt-Metallen, Pt-Legierungen, Au-Legierungen oder Sn-Legierungen zur Ausbildung der elektrolytisch nicht korrodierbaren Abschnitte (10);
c) Co-Basislegierungen zur Ausbildung der elektrolytisch korrodierbaren Stellen (11) mit Edelmetallen oder Edelmetall-Legierungen, vorzugsweise Pt, Pt-Metallen, Pt-Legierungen, Au-Legierungen oder Sn-Legierungen zur Ausbildung der elektrolytisch nicht korrodierbaren Abschnitte (10).

6. Modul nach einem der vorhergehenden Ansprüche, **dadurch gekennzeichnet, daß** die Formteile (17) an Mikrowindungen der Occlusionswendel (3) angepaßt sind.

7. Modul nach einem der vorhergehenden Ansprüche, **dadurch gekennzeichnet, daß** die Formteile (17) mit den nicht korrodierbaren Abschnitten (10) durch Löt- und/oder Klebe- und/oder Schweißvorgänge und/oder mechanische Verfahren verbunden sind.

8. Modul nach einem der vorhergehenden Ansprüche, **dadurch gekennzeichnet, daß** die Formteile (17) vorkorrodiert sind.

9. Modul nach einem der vorhergehenden Ansprüche, **dadurch gekennzeichnet, daß** die Formteile (17) teilweise mit einem Material beschichtet sind, welches in der Spannungsreihe über dem die Formteile (17) bildenden Material steht.

10. Modul nach Anspruch 9, **dadurch gekennzeichnet, daß** die Formteile (17) aus nicht rostendem Stahl bestehen und die Beschichtung aus Zn, Zn-Legierungen, Sn oder Sn-Legierungen besteht.

11. Modul nach einem der vorhergehenden Ansprüche, **dadurch gekennzeichnet, daß** die Formteile (17) Mikrosystembausteine (16) sind.

12. Modul nach Anspruch 11, **dadurch gekennzeichnet, daß** die Mikrosystembausteine Mikroablösestellen (24) als elektrolytisch korrodierbare Stellen (11) enthalten.

13. Modul nach einem der vorhergehenden Ansprüche, **dadurch gekennzeichnet, daß** der Durchmesser der elektrolytisch korrodierbaren Formteile (17) zwischen 0,01 bis 0,05 mm, vorzugsweise 0,02 bis 0,04 mm und besonders bevorzugt 0,03 mm beträgt.

14. Elektrolytisch korrodierbares Formteil für die Verbindung von elektrolytisch nicht korrodierbaren Abschnitten (10) von zur Implantation in Blutgefäße, insbesondere Aneurysmen, bestimmten Occlusionswendeln (3), **dadurch gekennzeichnet, daß** das Formteil (17) vorkorrodiert ist.

15. Formteil nach Anspruch 14, **dadurch gekennzeichnet, daß** es aus nicht rostendem Stahl besteht.

16. Formteil nach Anspruch 15, **dadurch gekennzeichnet, daß** nicht rostender Stahl der Typen AISI 301, 304 oder 316 oder Untergruppen verwandt ist.

17. Formteil nach einem der Ansprüche 14 bis 16, **dadurch gekennzeichnet, daß** es an Mikrowindungen der Occlusionswendeln (3) angepaßt ist.

18. Formteil nach einem der Ansprüche 14 bis 17, **dadurch gekennzeichnet, daß** es teilweise mit einem Material beschichtet ist, welches in der Spannungsreihe über dem das Formteil (17) bildenden Material steht.

19. Formteil nach Anspruch 18, **dadurch gekennzeichnet, daß** das Formteil (17) aus nicht rostendem Stahl besteht und die Beschichtung aus Zn, Zn-Legierungen, Sn oder Sn-Legierungen.

20. Formteil nach einem der Ansprüche 14 bis 19, **dadurch gekennzeichnet, daß** das Formteil (17) als Mikrosystembaustein (16) ausgebildet ist.

21. Formteil nach dem vorhergehenden Anspruch, **dadurch gekennzeichnet, daß** der Mikrosystembaustein eine Mikroablösestelle (24) als elektrolytisch korrodierbare Stelle (11) enthält.

22. Formteil nach einem der Ansprüche 14 bis 21, **dadurch gekennzeichnet, daß** der Durchmesser des elektrolytisch korrodierbaren Formteils (17) zwischen 0,01 und 0,05 mm, vorzugsweise 0,02 und 0,04 mm und besonders bevorzugt 0,03 mm beträgt.

## Claims

1. Module, which is suited to form an occlusion coil (3) for the implantation into blood vessels, particularly aneurysms, by combination with further modules, the module being composed of electolytically non-corrodible sections (10) and electolytically corrodible sites (11) joined together, the electolytically corrodible sites (11) being constituted by fittings (17) and the fittings (17) and the electolytically non-corrodible sections (10) being composed by combinations of materials forming local elements at both sites of the fittings (17).

2. Module as set forth in claim 1, **characterized in that** the electolytically non-corrodible sections (10) comprise noble metals or noble metal alloys, preferably platinum metal alloys.

3. Module as set forth in claims 1 or 2, **characterized in that** the fittings (17) comprise non-noble metals or alloys thereof preferably stainless steel.

4. Module a set forth in claim 3, **characterized in that** stainless steel of the types AISI 301, 304 or 316 or, respectively subgroups, is employed for the formation of the electrolytically corrodible sites (11).

5. Module as set forth in one of the proceeding claims 1 to 3, **characterized in that** the following material combinations are utilized:
a) Stainless steel, preferably of the type AISI 301, 304 or 316 or subgroups for the formation of the electrolytically corrodible sites (11) with noble metals or noble metal alloys, preferably Pt or Pt metals, PT alloys, Au alloys or Sn alloys for the formation of the electrolytically corrodible sections (10);
b) Ti or TiNi alloys for the formation of electrolytically corrodible sites (11) with noble metals or noble metal alloys, preferably Pt, Pt metals, Pt alloys, Au alloys or Sn alloys for the formation of the electrolytically corrodible sections (10);
c) Co based alloys for the formation of the electrolytically corrodible sites (11) with noble metals or noble metal alloys, preferably Pt, Pt metals, Pt alloys, Au alloys or Sn alloys for the formation of the electolytically non-corrodible sections (10).

6. Module as set forth in one of the preceding claims, **characterized in that** the fittings (17) are adapted to micro-windings of the occlusion coil (3).

7. Module as set forth in one of the preceding claims, **characterized in that** the fittings (17) are joined to the non-corrodible sections (10) by soldering and/or adhesive and/or welding operations and/or mechanical methods.

8. Module as set forth in one of the preceding claims, **characterized in that** the fittings (17) are pre-corroded.

9. Module as set forth in one of the preceding claims, **characterized in that** the fittings (17) are partly coated with a material, which is higher up in the electrochemical series than the material forming the fittings (17).

10. Module as set form in claim 9, **characterized in that** the fittings (17) consist of stainless steel and the coating consists of Zn, Zn alloys, Sn or Sn alloys.

11. Module as set forth in one of the preceding claims, **characterized in that** the fittings (17) are micro-system components (16).

12. Module as set forth in claim 11, **characterized in that** the micro-system components comprise micro-severance sites (24) as electrolytically, corrodible sites (11).

13. Module as set forth in one of the preceding claims, **characterized in that** the diameter of the electolytically corrodible fittings (17) is between 0.01 and 0.05 mm, preferably 0.02 and 0.04 mm and particularly 0.03 mm.

14. Electrolytically corrodible fitting for the connexion of electolytically non-corrodible sections (10) of occlusion coils (3) designated for the implantation into blood vessels, in particular aneurysms, **characterized in that** the fitting (17) is pre-corroded.

15. Fitting as set forth in claim 14, **characterized in that** it consist of stainless steel.

16. Fitting as set forth in claim 15, **characterized in that** stainless steel of the types AISI 301, 304 or 316 or subgroups is utilized.

17. Fitting as set forth in one of claims 14 to 16, **characterized in that** is adapted to micro-windings of the occluding coils (3).

18. Fitting as set forth in one of claims 14 to 17, **characterized in that** it is partly coated with a material which is higher up in the electrochemical series than the material forming the fitting (17).

19. Fitting as set forth in claim 18, **characterized in that** the fitting (17) consist of stainless steel and the coating is from Zn, Zn alloys, Sn or Sn alloys.

20. Fitting as set forth in one of claims 19, **characterized in that** the fitting (17) is designed as a micro-system component (16).

21. Fitting as set forth in the precedings claim, **characterized in that** the micro-system component (16) comprises a micro-severance site (2) as electrolytically corrodible site (11).

22. Fitting as set forth in one of claims 14 to 21, **characterized in that** the diameter of the electrolytically corrodible fitting (17) is between 0.01 and 0.05 mm, preferably 0.02 and 0.04 mm and particularly 0.03 mm.

## Revendications

1. Module, qui convient pour former, par assemblage avec d'autres modules, une spire anti-occlusion **(3)** destinée à une implantation dans des vaisseaux sanguins, en particulier des anévrysmes, ce module se composant de sections non corrodables électrolytiquement **(10),** assemblées les unes aux autres, et de parties **(11)** corrodables électrolytiquement ; les parties **(11)** corrodables électrolytiquement se présentant sous forme de pièces moulées **(17),** et les pièces moulées **(17)** et les sections **(10)** non corrodables électrolytiquement se composant de combinaisons de matériaux qui forment des éléments locaux des deux côtés de la pièce moulée **(17).**

2. Module selon la revendication 1, **caractérisé en ce que** les sections **(10)** non corrodables électrolytiquement contiennent des métaux nobles ou des alliages de métaux nobles, de préférence des alliages de métaux à base de platine.

3. Module selon la revendication 1 ou 2, **caractérisé en ce que** les pièces moulées **(17)** contiennent des métaux non nobles ou des alliages de ceux-ci, de préférence de l'acier inoxydable.

4. Module selon la revendication 3, **caractérisé en ce que** l'on utilise un acier inoxydable des types AISI 301, 304 ou 316 ou des sous-groupes pour réaliser les parties **(11)** corrodables électrolytiquement.

5. Module selon l'une des revendications précédentes 1 à 3, **caractérisé en ce que** l'on utilise les combinaisons de matériaux suivantes :
a) aciers inoxydables, de préférence du type AISI 301, 304, 316 ou leurs sous-groupes pour la réalisation des parties **(11)** corrodables électrolytiquement avec des métaux nobles ou des alliages de métaux nobles, de préférence Pt ou des métaux à base de Pt, alliages de Pt, alliages d'or ou alliages de Sn pour la réalisation des sections **(10)** non corrodables électrolytiquement ;
b) Ti ou alliages de TiNi pour la réalisation des parties (11) corrodables électrolytiquement avec des métaux nobles ou des alliages des métaux nobles, de préférence Pt ou des métaux à base de Pt, alliages de Pt, alliages d'Au ou alliages de Sn pour la réalisation des sections (10) non corrodables électrolytiquement ;
c) alliages à base de Co pour la réalisation des parties **(11)** corrodables électrolytiquement avec des métaux nobles ou des alliages des métaux nobles, de préférence Pt ou des métaux à base de Pt, alliages de Pt, alliages d'Au ou alliages de Sn pour la réalisation des sections **(10)** non corrodables électrolytiquement.

6. Module selon l'une des revendications précédentes, **caractérisé en ce que** les pièces moulées **(17)** sont adaptées sur des micro-enroulements de la spire anti-occlusion **(3).**

7. Module selon l'une des revendications précédentes, **caractérisé en ce que** les pièces moulées **(17)** sont reliées aux sections **(10),** non corrodables électrolytiquement, par des opérations de brasage et/ou collage et/ou soudage et/ou des procédés mécaniques.

8. Module selon l'une des revendications précédentes, **caractérisé en ce que** les pièces moulées **(17)** sont pré-corrodées.

9. Module selon l'une des revendications précédentes, **caractérisé en ce que** les pièces moulées **(17)** sont partiellement revêtues d'un matériau qui, dans la série des tensions, se trouve au-dessus du matériau formant les pièces moulées **(17).**

10. Module selon la revendication 9, **caractérisé en ce que** les pièces moulées **(17)** sont constituées d'acier inoxydable et le revêtement est constitué de Zn, d'alliages de Zn, de Sn ou d'alliages de Sn.

11. Module selon l'une des revendications précédentes, **caractérisé en ce que** les pièces moulées **(17)** sont des composants de microsystèmes **(16).**

12. Module selon la revendication 11, **caractérisé en ce que** les composants de microsystèmes contiennent des micro-parties de décollement **(24)** en tant que parties **(11)** corrodables électrolytiquement.

13. Module selon l'une des revendications précédentes, **caractérisé en ce que** le diamètre des pièces moulées **(17)** corrodables électrolytiquement vaut de 0,01 à 0,05 mm, de préférence 0,02 à 0,04 mm, et de manière particulièrement préférée 0,03 mm.

14. Pièce moulée corrodable électrolytiquement pour la liaison de sections **(10)** non corrodables électrolytiquement de spires anti-occlusion **(3)** destinées à une implantation dans des vaisseaux sanguins, en particulier des anévrysmes, **caractérisée en ce que** la pièce moulée **(17)** est pré-corrodée.

15. Pièce moulée selon la revendication 14, **caractérisée en ce qu'**elle est constituée d'acier inoxydable.

16. Pièce moulée selon la revendication 15, **caractérisée en ce que** l'on utilise un acier inoxydable des types AISI 301, 304 ou 316 ou des sous-groupes de ceux-ci.

17. Pièce moulée selon l'une des revendications 14 à 16, **caractérisée en ce qu'**elle est adaptée à des micro-enroulements des spires anti-occlusion **(3).**

18. Pièce moulée selon l'une des revendications 14 à 17, **caractérisée en ce qu'**elle est revêtue partiellement d'un matériau qui, dans la série des tensions, se trouve au-dessus du matériau formant la pièce moulée **(17).**

19. Pièce moulée selon la revendication 18, **caractérisée en ce que** la pièce moulée **(17)** est constituée d'acier inoxydable et le revêtement est constitué de Zn, d'alliages de Zn, de Sn ou d'alliages de Sn.

20. Pièce moulée selon l'une des revendications 14 à 19, **caractérisé en ce que** la pièce moulée **(17)** est conformée en composants de microsystème **(16).**

21. Pièce moulée selon la revendication précédente, **caractérisée en ce que** le composant de microsystème contient une micro-partie de décollement **(24)** en tant que partie **(11)** corrodable électrolytiquement.

22. Pièce moulée selon l'une des revendications 14 à 21, **caractérisée en ce que** le diamètre de la pièce moulée **(17)** corrodable électrolytiquement vaut de 0,01 à 0,05 mm, de préférence 0,02 à 0,04 mm, et de manière particulièrement préférée 0,03 mm.
